# FASCICULE DE BREVET EUROPEEN

(11) **EP 0 702 010 B1**
(45) Date de publication et mention de la délivrance du brevet: **15.03.2000**
(21) Numéro de dépôt: 95402076.4
(22) Date de dépôt: 14.09.1995
(51) Int. Cl.: C07D 311/66, C07D 307/81, C07D 311/58, C07D 311/16, C07D 295/18, C07D 295/08

(54) **Nouveaux dérivés du benzopyrane, leur procédé de préparation et les compositions pharmaceutiques qui les contiennent**
Benzopyranverbindungen, Verfahren zu ihrer Herstellung und diese enthaltende pharmazeutischen Zubereitungen
Benzopyranderivatives, process for their preparation and pharmaceutical compositions containing them

(30) Priorité: 15.09.1994 FR 9410987
(43) Date de publication de la demande: 20.03.1996
(73) Titulaire: ADIR ET COMPAGNIE, 92415 Courbevoie Cédex (FR)
(72) Inventeur: Baziard-Mouysset, Geneviève, Faculté de Pharmacie, F-31400 Toulouse (FR); Younes, Sallouma, Faculté de Pharmacie, F-31400 Toulouse (FR); Labssita, Youssef, Faculté de Pharmacie, F-31400 Toulouse (FR); Payard, Marc, F-31130 Balma (FR); Caignard, Daniel-Henri, F-75015 Paris (FR); Renard, Pierre, F-78000 Versailles (FR); Rettori, Marie-Claire, F-92400 Courbevoie (FR)

(56) Documents cités:
- EP-A- 0 076 996
- DE-A- 2 157 424

## Description

La présente invention concerne de nouveaux dérivés du benzopyrane ou du dihydrobenzofurane à structure pipérazinique ainsi que leur procédé de préparation et les compositions pharmaceutiques qui les contiennent.

L'art antérieur décrit des composés à structure arylalkylpipérazino cétobenzofuranique (DE 2157424) utiles en tant qu'antidépresseurs ou anti-inflammatoires et dépourvus des effets secondaires associés.

On connaît par ailleurs dans la littérature des amino méthyl chromannes (Indian Journal of Chemistry, 1981, 20 B : 1063 - 1067 et 1982, 21B : 344 - 347). Les seuls dérivés de structure pipérazinique décrits dans ces documents sont des dérivés de la phényl pipérazine. Certains sont mentionnés comme doués de propriétés dépressives du système nerveux central. Une recherche du mécanisme de leur activité a montré que ces composés de structure phénylpipérazine possédaient une forte affinité sur les récepteurs de la sérotonine et de la dopamine. L'activité centrale de telles substances s'accompagne d'effets secondaires importants tels que, parmi les plus fréquents, sédation, somnolence ou, plus graves, accidents aigus paroxystiques : tels que spasmes buccolinguaux et crises oculogyres fort génants pour le malade.

Les composés de la présente invention, possédant une structure arylalkyl pipérazino alkyl benzopyranne ainsi que leurs dérivés, sont par contre dépourvus d'affinité à la fois pour les récepteurs de la sérotonine et de la dopamine mais possédent une affinité sélective et de très haut niveau pour les récepteurs sigma. Ce profil rend les composés de l'invention utiles dans les troubles du système nerveux central, en étant dépourvus des effets secondaires rencontrés chez les produits de l'art antérieur. Les composés de l'invention sont également utiles dans la prévention et le traitement des maladies impliquant les récepteurs sigma. En particulier, ils sont utiles dans la prévention et le traitement des maladies circulatoires cérébrales, des troubles de la mémoire et de la maladie d'Alzheimer, des maladies inflammatoires d'origine immunitaire telles que l'arthrite, et des troubles du péristaltisme intestinal.
Plus particulièrement, la présente invention concerne les dérivés de formule (I) : 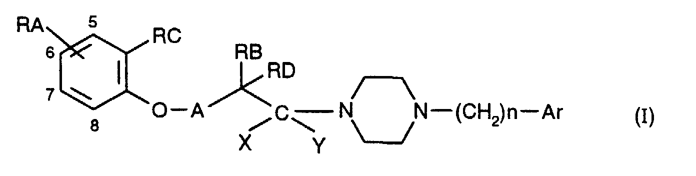 dans laquelle
- Ar représente un groupe phényle, naphtyle, phényle substitué ou naphtyle substitué,
- n représente un entier compris inclusivement entre 1 et 4,
   . RB représente un groupement alkyle, et dans ce cas A représente une liaison simple et RA et RC, identiques ou différents, représentent indépendamment l'un de l'autre un atome d'hydrogène ou un groupement choisi parmi halogène, alkyle, alkyle substitué par un ou plusieurs halogènes, et alkoxy,
   . ou RB et RC forment ensemble un pont -(CH₂)_{q}- avec q valant 0, 1 ou 2 et dans ce cas A représente un pont -(CH₂)ₚ- avec p représentant 0, 1 ou 2 et tel que p + q = 1 ou 2, et dans ce cas RA représente un groupement hydroxy ou alkoxy situé en position 5 du noyau aromatique qui le porte ou RA représente un atome d'hydrogène ou d'halogène, en position quelconque du cycle aromatique,
   . ou RB et RC forment ensemble un pont -CH=, et la liaison qui le lie au cycle aromatique est simple, et dans ce cas A représente un groupement CH₂ et RA représente un atome d'hydrogène, un groupement hydroxy ou alkoxy situé en position 5 du cycle aromatique qui le porte,
   . ou RB et RC forment ensemble une liaison simple et alors A représente un groupement 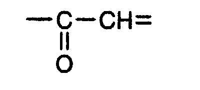 le carbonyle étant lié à l'oxygène et la liaison joignant A au carbone porteur de la chaîne latérale est double, et dans ce cas RA représente un atome d'hydrogène ou un groupement hydroxy ou alkoxy,
- lorsque RB représente un groupement alkyle, X et Y représentent chacun deux atomes d'hydrogène ou forment ensemble avec l'atome de carbone qui les porte un groupement C=O, et RD représente un atome d'hydrogène ou un groupement alkyle,
- lorsque RB et RC forment un pont, X et Y représentent chacun deux atomes d'hydrogène et RD, qui n'existe que lorsque toutes les liaisons du carbone qui le porte sont simples, représente un atome d'hydrogène,
étant entendu que, sauf mention contraire,
- les termes "alkyle" et "alkoxy" désignent des groupements saturés linéaires ou ramifiés contenant de 1 à 6 atomes de carbone,
- le terme "substitué" affectant les substituants phényle et naphtyle signifie que ceux-ci peuvent être substitués par un à trois groupements choisis parmi hydroxy, alkyle, alkyle substitué par un ou plusieurs halogènes, alkoxy, et halogène,
leurs isomères optiques sous forme pure ou sous forme de mélange, et leurs sels d'addition à un acide ou une base pharmaceutiquement acceptable.

L'invention concerne particulièrement les composés de formule (I) dans laquelle, pris ensemble ou séparément,
- RA représente un atome d'hydrogène,
- RA représente un atome d'halogène,
- RA représente un groupement hydroxy,
- RA représente un groupement alkoxy,
- Ar représente un phényle non substitué,
- Ar représente un phényle substitué,
- Ar représente un phényle substitué par un groupement alkoxy,
- Ar représente un naphtyle,
- RB représente un groupement alkyle et RC représente un atome d'hydrogène,
- n est égal à 1,
- RD représente un hydrogène,
- et RD représente un méthyle.

De façon préférentielle, l'invention concerne :
. les composés de formule (Ic), cas particulier des composés de formule (I) pour lesquels RB et RC forment un pont -(CH₂)_{q} avec q valant 2, et RD représente un atome d'hydrogène 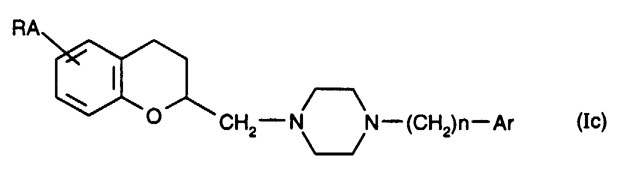 où RA, n et Ar ont la même définition que dans la formule (I),
   leurs isomères optiques sous forme pure ou sous forme de mélange ainsi que leurs sels d'addition à un acide ou à une base pharmaceutiquement acceptable,
. les composés de formule (Id), cas particulier des composés de formule (I) pour lesquels RB et RC forment un pont -(CH₂)_{q}- avec q valant 1, A représente CH₂, et RD un atome d'hydrogène, 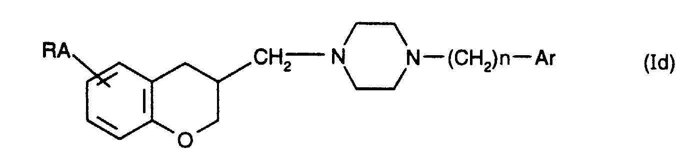 où RA, n et Ar ont la même définition que dans la formule (I),
   leurs isomères optiques sous forme pure ou sous forme de mélange ainsi que leurs sels d'addition à un acide ou à une base pharmaceutiquement acceptable,
. les composés de formule (Ie), cas particulier des composés de formule (I) pour lesquels RB et RC forment un pont -CH= , la liaison qui le lie au carbone porteur de la chaîne latérale est double, et A représente un groupement -CH₂-, 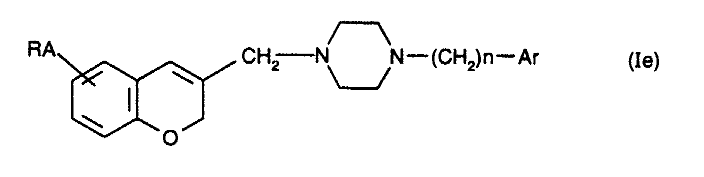 où RA, n et Ar ont la même définition que dans la formule (I),
   leurs isomères optiques sous forme pure ou sous forme de mélange ainsi que leurs sels d'addition à un acide ou à une base pharmaceutiquement acceptable,
. les composés de formule (If), cas particulier des composés de formule (I) pour lesquels RB et RC représentent une liaison simple et A représente un chaînon -CO-CH=, le carbonyle étant lié à l'oxygène, 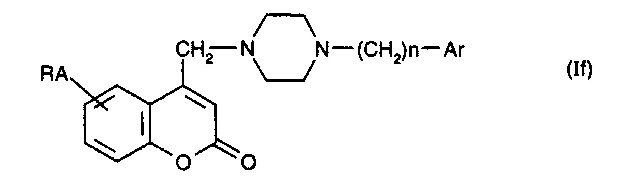 où RA, n et Ar ont la même définition que dans la formule (I),
   leurs isomères optiques sous forme pure ou sous forme de mélange et leurs sels d'addition à un acide ou à une base pharmaceutiquement acceptable,
. les composés de formule (Ig), cas particulier des composés de formule (I) pour lesquels RB représente un groupement alkyle inférieur, 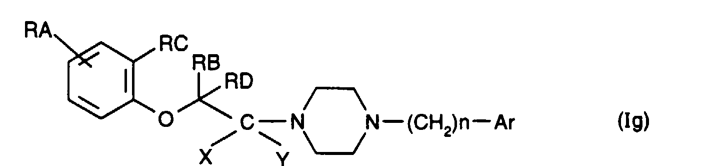 où RA, RC, RD, X, Y, n, et Ar ont la même définition que dans la formule (I),
   leurs isomères optiques sous forme pure ou sous forme de mélange et leurs sels d'addition à un acide ou à une base pharmaceutiquement acceptable,
. les composés de formule (Ih), cas particulier des composés de formule (I) pour lesquels RB et RC forment un pont -(CH₂)_{q}- avec q valant 1, RD représente un atome d'hydrogène, A représente un groupement -(CH₂)ₚ- avec p valant 0, 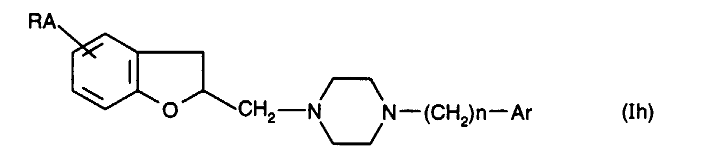 où RA, n, et Ar ont la même définition que dans la formule (I),
   leurs isomères optiques sous forme pure ou sous forme de mélange et leurs sels d'addition à un acide ou à une base pharmaceutiquement acceptable.

De façon particulière, les radicaux alkyles présents dans la formule (I) peuvent être choisis parmi méthyle, éthyle, n-propyle, isopropyle, n-butyle, isobutyle, sec-butyle, tert-butyle, pentyle et hexyle.

Les radicaux alkoxy présents dans la formule (I) penvent être choisis parmi méthoxy, éthoxy, n-propoxy, isopropoxy, n-butoxy, isobutoxy, sec-butoxy, tert-butoxy, pentyloxy, et hexyloxy.

Les halogènes présents dans la formule (I) peuvent être choisis parmi le brome, le chlore, le fluor, et l'iode.

L'invention concerne également particulièrement les composés de formule (I) qui sont :
- la 1-[(5-fluoro-2,3-dihydrobenzofur-2-yl)méthyl]-4-benzylpipérazine,
- la 1-[(2,3-dihydrobenzofur-2-yl)méthyl]-4-benzylpipérazine,
- la 1-[(chroman-2-yl)méthyl)]-4-benzylpipérazine,
- la 1-[(7-méthoxy-[2H]-2-oxochrom-3-èn-4-yl)méthyl]-4-benzylpipérazine,
- la 1-[(7-méthoxy-[2H]-2-oxochrom-3-èn-4-yl)méthyl]-4-(4-méthoxybenzyl)pipérazine,
- la 1-[2-(phénoxy)propyl]-4-benzylpipérazine,
leurs isomères optiques sous forme pure ou sous forme de mélange et leurs sels d'addition à un acide pharmaceutiquement acceptable.

L'invention s'étend également au procédé de préparation des composés de formule (I), caractérisé en ce que l'on fait réagir un composé de formule (II) : 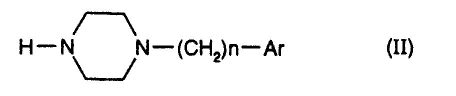 dans laquelle Ar et n sont tels que définis dans la formule (I),
- soit avec un composé de formule (III) : 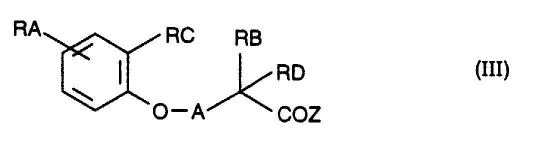 dans laquelle RA, RB, RC, RD et A sont tels que définis dans la formule (I), Z représentant un groupement hydroxy ou un atome de chlore,
   pour obtenir un composé de formule (Ia) : 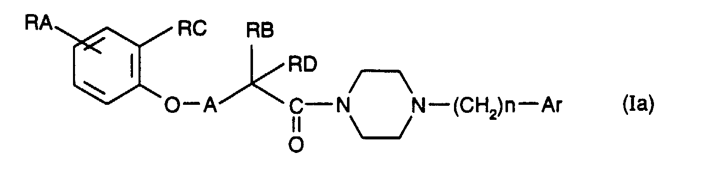 dans laquelle RA, RB, RC, RD, Ar, n et A sont tels que définis précédemment, lequel composé (Ia) peut être soumis, en fonction de la structure du composé de formule (I) que l'on souhaite obtenir, à une réduction pour donner un composé de formule (Ib) : 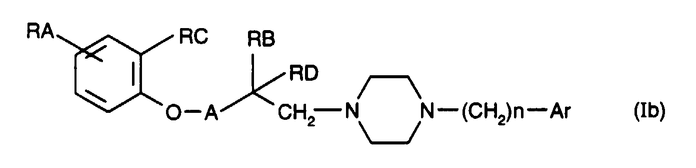 où RA, RB, RC, RD, Ar, n et A ont la même définition que précédemment,
- soit avec un composé de formule (IV) en présence d'un agent alcalin : 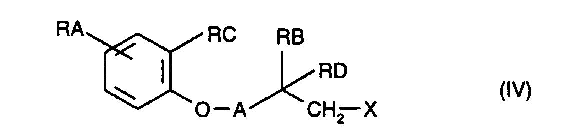 dans laquelle RA, RB, RC, RD et A sont tels que définis précédemment et X représente un atome d'halogène, pour obtenir un composé de formule (Ib) telle que définie précédemment,
   les composés de formule (Ia) et (Ib) formant l'ensemble des composés de formule (I) pouvant être séparés en leurs différents isomères optiques sous forme pure ou sous forme de mélange et salifiés avec un acide ou une base pharmaceutiquement acceptable.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour former un sel d'addition avec les composés de l'invention, on peut citer à titre d'exemples et de façon non limitative la soude, la potasse, la triéthylamine, la diéthylamine, l'éthanolamine, l'arginine, la lysine, et la diéthanolamine.

Les matières premières utilisées dans le procédé précédemment décrit sont soit commerciales, soit aisément accessibles à l'homme du métier selon des procédés connus dans la littérature.

Les chlorures d'acides, s'ils ne sont pas commerciaux, sont obtenus par traitement des acides correspondants avec un agent de chloration comme le chlorure de thionyle.

Les composés de formule (I) possèdent des propriétés pharmacologiques intéressantes.

La très haute affinité des dérivés de l'invention pour les récepteurs a (sigma) les rend utilisables dans le traitement des désordres moteurs tels que les dystonies (Walker JM. Drug specificity of pharmacology dystonia, Pharmacol. Biochem. Behav. 1990, 36 : 151), la dyskinésie tardive (Lindstrom L.H. Acta Psychiatr. Scand. 1988, 77 : 1122), les troubles psychotiques (Chouinard F., Annable L. Psychopharmacology 1984, 84 : 282), et des désordres tels que les dommages liés à l'ischémie, l'insuffisance circulatoire cérébrale, les troubles de mémoire, la maladie d'Alzheimer et les états de choc (Pontecorvo M.J. Brain Res. Bull. 1991, 26 : 461), la régulation des phénomènes immunitaires (Carroll F.I. Med. Chem. Re. 1992, 2 : 3), le traitement des toxicomanies à la cocaïne (Abou - Gharbia M., Academic. Press. (Inc. Bristol. J. Ed. Publisher) 1993, 28 : 1), le diagnostic et la localisation des tumeurs (Hudzick T.J., Psychopharmacology. 1992, 108 : 115 ; Abou - Gharbia M. Academic. Press. (Inc. Bristol. J. Ed. Publisher) 1993, 28 : 1), le traitement des vomissements (Hudzick, T.J., Eur. J. Pharmacol. 1993, 236 : 279), les maladies inflammatoires d'origine immunitaire comme l'arthrite, l'inflammation bronchopulmonaire et le psoriasis, les pathologies allergiques, l'eczéma, le choc septique, ainsi que les troubles de la motricité intestinale. Pour ce qui concerne le traitement des troubles du système nerveux central, les composés de l'invention sont dépourvus d'affinité pour les récepteurs centraux autres que le récepteur σ, et sont donc dépourvus des effets secondaires classiquement rencontrés dans ce genre d'activité.

La présente invention a également pour objet les compositions pharmaceutiques contenant les composés de formule (I) ou un de leurs sels d'addition à un acide ou une base pharmaceutiquement acceptable, en combinaison avec un ou plusieurs excipients.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon l'âge, le sexe, et le poids du patient, la voie d'administration, la nature de l'indication thérapeutique, ou des traitements éventuellement associés et s'échelonne entre 0,1 mg et 1 g par 24 heures en 1 ou 2 prises, plus particulièrement entre 1 et 100 mg, par exemple entre 1 et 10 mg.

Les exemples suivants illustrent l'invention, mais ne la limitent en aucune façon.

Les spectres de résonance magnétique nucléaire ¹H ont été réalisés en utilisant le TMS (tétraméthylsilane) comme référence interne. Les déplacements chimiques sont exprimés en partie par million (p.p.m). Les spectres infrarouge ont été effectués sous forme de pastille de bromure de potassium renfermant environ 1 % du produit à analyser.

### EXEMPLE 1 : 1-[(5-FLUORO-2,3-DIHYDROBENZOFUR-2-YL)METHYL]-4-BENZYLPIPERAZINE

On met en solution 0,03 mole de la 1-[(5-fluoro-2,3-dihydrobenzofur-2-yl)carbonyl]-4-benzylpipérazine (résultant de la condensation de l'acide 2-(5-fluoro-2,3-dihydrobenzofuryl)carboxylique avec la N-benzyl pipérazine) dans 150 ml de tétrahydrofurane (T.H.F.) anhydre. On ajoute deux fois 0,03 mole d'hydrure d'aluminiumlithium sous forme de pellets. Le mélange est agité à température ambiante. On obtient le composé du titre.

### Caractéristiques :

Masse moléculaire pour C₂₀H₂₃FN₂O, 2HCl : 399 g.mol⁻¹
Rendement : 85 %
Rf : 0,66 (Ethanol)
Point de fusion : 214°C

### Spectroscopie dans l'infra-rouge (KBr, νcm⁻¹) :

3140 - 2800 (CH, CH₂) ; 2550 - 2440 (NH⁺) ; 1620 - 1600 (C=C).

### Résonnance magnétique nucléaire ¹H(D.M.S.O-d₆, δ ppm) :

3,01 (2dd, 2H, CH₂-CH-O : J1 = 9,00 Hz et J2 = 15,87 Hz) 3,78 - 3,45 (m, 12 H, CH₂-N⁺, ⁺N-CH₂-Ar et 8H pipérazine) ; 5,43 (m, 1H, CH₂-CH-O) ; 7,77 - 6,84 (m, 8H, aromatiques).

### EXEMPLE 2 : 1-[(CHROMAN-2-YL)METHYL]-4-BENZYLPIPERAZINE

### Stade A : 1-[(chroman-2-yl)carbonyl]-4-benzylpipérazine

Dans un erlenmeyer contenant une solution de 0,01 mole de 1-benzylpipérazine dans 100 ml de chlorure de méthylène, on introduit une solution de 0,01 mole de chlorure de l'acide chroman-2-carboxylique dans 100 ml de chlorure de méthylène. Le mélange est agité pendant 5 heures à température ambiante. La réaction est suivie par chromatographie sur couche mince de silice. Après filtration, le produit du titre est obtenu sous forme de chlorhydrate et est recristallisé dans l'éthanol.

### Caractéristiques :

Masse moléculaire pour C₂₁H₂₄N₂O₂,HCl : 372,5 g.mol⁻¹
Rendement : 52 %
Rf : 0,77 (Acétate d'éthyle)
Point de fusion : 230°C

### Spectroscopie dans l'infra-rouge (KBr, νcm⁻¹) :

3150, 3090, 3035, 2995, 2810 (CH, CH₂) ; 2600 - 2400 (NH⁺) ; 1650 (CO) ; 1610 (C=C).

### Résonnance magnétique nucléaire ¹H (CDCl₃, δ ppm) :

2,42(m, 2H, CH₂-CH₂-CH) ; 2,51(m, 4H, pipérazine) ; 2,89(t, 2H, CH₂-CH₂-CH) ; 3,52(s, 2H, N-CH₂-Ar) ; 3,82(m, 4H, pipérazine) ; 4,74(m, 1H, O-CH-O) ; 6,80 - 7,34(m, 9H, aromatiques).

### Stade B : 1-[(chroman-2-yl)méthyl]-4-benzylpipérazine

En procédant de la même façon que pour l'exemple 1, mais en partant du chlorhydrate de la 1 -[(chroman-2-yl)carbonyl]-4-benzylpipérazine obtenu au stade A, on obtient le composé du titre.

### Caractéristiques :

Masse moléculaire pour C₂₁H₂₆N₂O, 2HCl : 395 g.mol⁻¹
Rendement : 93 %
Rf : 0,55 (Ethanol)
Point de fusion : > 230°C

### Spectroscopie dans l'infra-rouge (KBr, νcm⁻¹) :

3210, 3000, 2980 (CH, CH₂) ; 2700 - 2200 (NH⁺) ; 1605, 1590 (C=C).

### Résonnance magnétique nucléaire ¹H (D.M.S.O-d₆, δ ppm) :

1,69 - 2,96 (m, 14H, CH₂-CH₂-CH-O, CH₂-N, pipérazine) ; 3,46 (s, 2H, N-CH₂) ; 4,26 (m, 1H, CH₂-CH₂-CH-O) ; 6,79 - 7,47 (m, 9H, aromatiques). Les 2H⁺ ne sont pas visibles parce que le produit et le D.M.S.O sont hygroscopiques, ce qui implique un échange avec l'eau.

### EXEMPLE 3 : 1-[(7-METHOXY-2-OXOCHROM-3-EN-4-YL)METHYL]-4-BENZYLPIPERAZINE (base et chlorhydrate)

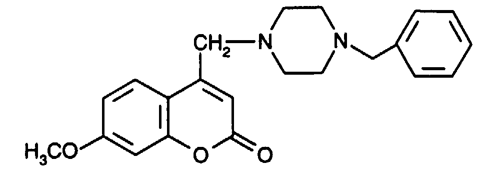

Dans un erlenmeyer contenant 150 ml de tétrahydrofurane, on introduit 0,03 mole de 1-benzylpipérazine, 0,03 mole de 7-méthoxy 2-oxo 4-(bromométhyl)chrom-3-ène et 0,03 mole de bicarbonate de potassium. Le mélange est porté à reflux pendant 24 heures. La réaction est terminée et après refroidissement, la solution est filtrée et évaporée sous pression réduite. Le résidu est alors purifié par chromatographie sur colonne de gel de silice en utilisant généralement le chlorure de méthylène comme éluant. On obtient ensuite le produit final sous forme de base.

### Caractéristiques :

Masse moléculaire pour C₂₂H₂₄N₂O₃, 2HCl : 437 g.mol⁻¹
Rendement : 80 %
Rf : 0,62 (Ethanol)
Point de fusion : 246°C

### Spectroscopie dans l'infra-rouge (KBr, νcm⁻¹) :

3010, 3000, 2980, 2965 (CH, CH₂, CH₃) ; 2650 - 2250 (NH⁺) ; 1600 (C=C).

### Résonnance magnétique nucléaire ¹H(D.M.S.O-d₆, δ ppm) :

3,15 (m, 8H, pipérazine) ; 3,95 (s, 2H, CH₂-N) ; 3,89 (s, 3H, O-CH₃) ; 4,32 (s, 2H, CH₂Ar) ; 6,45 (s, 1H, CO-CH) ; 6,92 - 7,87 (m, 8H, aromatiques) ; 11,2 (s large, 2H, 2NH).

### EXEMPLE 4 : 1-[(2,3-DIHYDROBENZOFUR-2-YL)METHYL]-4-BENZYLPIPERAZINE

En procédant de la même façon que dans l'exemple 1, mais en partant de 0,03 mole de 2-bromométhyl 2,3-dihydrobenzofurane, on obtient le composé du titre.

### Caractéristiques :

Masse moléculaire pour C₂₀H₂₄N₂O,2HCl : 381 g.mol⁻¹
Rendement : 82 %
Rf : 0,76 (Ethanol)
Point de fusion : 246°C

### Spectroscopie dans l'infra-rouge (KBr, ν cm⁻¹) :

3100, 3000, 2925 (CH, CH₂) ; 2650 - 2210 (N⁺H) ; 1595 (C=C)

### Résonnance magnétique nucléaire ¹H(D.M.S.O-d₆, δ ppm) :

3,02 (m, 2H, CH₂-CH-CH₂-N) ; 3,50 (m, 10 H, pipérazine et CH₂-CH-CH₂-N) ; 4,43 (s, N-CH₂-Ar) ; 5,33 (m, 1 H, CH₂-CH-CH₂-N) ; 6,88 - 7,70 (m, 9H, aromatiques).

### EXEMPLE 5 : 1-[(5-METHOXYCHROMAN-3-YL)METHYL]-4-BENZYLPIPERAZINE (base et chlorhydrate)

### Stade A : chlorhydrate de la 1-[(5-méthoxychroman-3-yl)carbonyl]-4-benzyl pipérazine

En procédant comme dans l'exemple 2 (stade A) mais en remplaçant le chlorure de l'acide chroman-2-carboxylique par le chlorure de l'acide 5-méthoxychromanyl-3-carboxylique on obtient le produit du titre.

### Caractéristiques :

Masse moléculaire pour C₂₂H₂₆N₂O₃,HCl : 402,5 g.mol⁻¹
Rendement : 63 %
Rf : 0,80 (Ethanol)
Point de fusion : 237°C

### Spectroscopie dans l'Infra-rouge (KBr, ν cm⁻¹)

3000, 2916, 2836 (CH, CH₂, CH₃) ; 2668 - 2331 (NH⁺) ; 1635 (CO) ; 1603 (C=C)

### Résonnance magnétique nucléaire ¹H (D.M.S.O-d₆, δ ppm):

2,84 (m, 3H, Ar-CH₂ et CH-CO) ; 3,28 (m, 8H, CH₂N) ; 3,88 (s, 3H,OCH₃) ; 4,39 (m, 2H, OCH₂) ; 4,60 (m, 2H, CH₂-Ar) ; 6,51 - 7,72 (m, 8H, Ar) ; 11,64 (s, large, 1H, NH⁺).

### Stade B : 1-[(5-METHOXYCHROMAN-3-YL)METHYL]-4-BENZYLPIPERAZINE (base et chlorhydrate)

En procédant comme dans l'exemple 1 mais en remplaçant la 1-[(5-fluoro-2,3-dihydro benzofur-2-yl)carbonyl]-4-benzylpipérazine par le chlorhydrate de la 1-[(5-méthoxy chroman-3-yl)carbonyl]-4-benzylpipérazine obtenu au stade A, on obtient le produit du titre.

### Caractéristique:

Masse moléculaire pour C₂₂H₂₈N₂O₂ : 352 g.mol⁻¹
Rendement : 42 %
Rf : 0,60 (Acétate d'éthyle)

### Spectroscopie dans l'infra-rouge (KBr, ν cm⁻¹):

3020, 2980, 2900, 2800 (CH, CH₂, CH₃) ; 1600 (C=C)

### Résonance magnétique nucléaire ¹H (D.M.S.O-d₆, δ ppm):

2,82 - 3,20 (m, 13H, CH₂-N) ; 3,41 (s, 2H, N-CH₂-Ar) ; 3,89 (s, 3H, O-CH₃) ; 4,27 (m, 2H, CH₂-CH-CH₂-O) ; 7,31 - 6,37 (m, 8H, aromatiques)

### Chlorhydrate :

### Caractéristiques :

Masse moléculaire pour C₂₂H₂₈N₂O₂, 2HCl : 425 g.mol⁻¹
Rendement : 85 %
Rf : 0,79 (Ethanol)
Point de fusion : 240°C

### Spectroscopie dans l'infra-rouge (KBr, ν cm⁻¹):

3040, 2990, 2870, 2800 (CH, CH₂, CH₃) ; 2500 - 2400 (NH⁺) ; 1610 - 1600 (C=C).

### EXEMPLE 6 : 1-[(5-METHOXYCHROM-3-EN-3-YL)METHYL)]-4-BENZYL PIPERAZINE (Base et chlorhydrate)

### Stade A : chlorhydrate de la 1-[(5-méthoxychrom-3-èn-3-yl)carbonyl]-4-benzyl pipérazine

En procédant comme dans l'exemple 2 (stade A) mais en remplaçant le chlorure de l'acide chroman-2-carboxylique par le chlorure de l'acide 5-méthoxychrom-3-èn-3-carboxylique on obtient le produit du titre.

### Caractéristiques :

Masse moléculaire pour C₂₂H₂₄N₂O₃,HCl : 400,5 g.mol⁻¹
Rendement : 71 %
Rf : 0,57 (Acétate d'éthyle)
Point de fusion : 234°C

### Spectroscopie dans l'infra-rouge (KBr, νcm⁻¹):

3110, 3025, 2990, 2950 (CH, CH₂, CH₃) ; 2657 - 2461 (NH⁺) ; 1645 (CO) ; 1610 (C=C)

### Résonance magnétique nucléaire ¹H (D.M.S.O-d₆, δppm) :

3,42 (m, 8H, CH₂N) ; 3,81 (m, 3H, OCH₃) ; 4,31 (s, 2H, N-CH₂-Ar) ; 4,23 (s, 1 H, CH-C-CO) 4,74 (s, 2H, CH₂-O) ; 6,47 à 7,64 (m, 8H, Ar) ; 11,68 (s, large, 1H, NH⁺).

### Stade B : 1-[(5-méthoxychrom-3-èn-3yl)méthyl]-4-benzylpipérazine (Base et chlorhydrate)

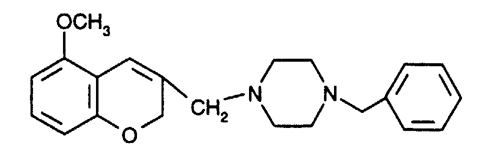

En procédant comme dans l'exemple 1 mais en remplaçant la 1-[(5-fluoro-2,3-dihydro benzofur-2-yl)carbonyl]-4-benzylpipérazine par le chlorhydrate de la 1-[(5-méthoxychrom-3-èn-3-yl)carbonyl]-4-benzylpipérazine au stade précédent, on obtient le produit du titre.

### Caractéristiques :

Masse moléculaire pour C₂₂H₂₆N₂O₂ 350 g.mol⁻¹
Rendement : 61 %
Rf : 0,69 (Acétate d'éthyle)
Point de fusion : 95°C

### Spectroscopie dans l'infra-rouge (KBr, νcm⁻¹) :

2970, 2930, 2810, 2795 (CH, CH₂, CH₃) ; 1610 (C=C)

### Résonnance magnétique nucléaire ¹H(D.M.S.O-d₆, δppm) :

2,46 (m, 8H, pipérazine) ; 3,06 (s, 2H, CH₂-N) ; 3,51 (s, 2H, CH₂-Ar) ; 3,80 (1s, 3H, O-CH₃) ; 4,70 (1s, 2H,O-CH₂) ; 6,39 - 7,32 (m, 9H, aromatiques et H₄).

### Chlorhydrate:

### Caractéristiques :

Masse moléculaire pour C₂₂H₂₆N₂O₂, 2HCl : 423 g.mol⁻¹
Rendement : 85 %
Rf : 0,80 (Ethanol)
Point de fusion : 216°C

### Spectroscopie dans l'infra-rouge (KBr, νcm⁻¹) :

2970, 2930, 2810, 2795 (CH, CH₂, CH₃) ; 1610 (C=C) ; 2550 - 2225 (NH⁺).

### EXEMPLE 7 : CHLORHYDRATE DE LA 1-(2-PHENOXYPROPIONYL)-4-BENZYLPIPERAZINE

En procédant comme dans l'exemple 2 (Stade A) mais en remplaçant le chlorure de l'acide chroman-2-carboxylique par le chlorure de l'acide 2-phénoxypropionique, on obtient le produit du titre.

### Caractéristiques :

Masse moléculaire pour C₂₀H₂₄N₂O₂, HCl : 360,5 g.mol⁻¹
Rendement : 82 %
Rf : 0,78 (Ethanol)
Point de fusion : 258°C

### Spectroscopie dans l'infra-rouge (KBr, νcm⁻¹) :

3075, 2990, 2950 (CH, CH₂, CH₃) ; 2600 - 2750 (NH⁺) ; 1665 (CO) ; 1610 (C=C).

### Résonnance magnétique nucléaire ¹H(D.M.S.O-d₆, δ ppm) :

3,47 (m, 10H, CH₂N et N-CH₂-Ar) ; 4,47 (m, 3H, CH₃) ; 5,37(m, 1H, O-CH-CO) ; 6,90 - 7,68 (m, 10H, Ar) ; 11,11 (s, large, H, NH⁺).

### EXEMPLE 8 : CHLORHYDRATE DE LA 1-(2-PHENOXYBUTYRYL)-4-BENZYLPIPERAZINE

En procédant comme dans l'exemple 2 (stade A)mais en remplaçant le chlorure de l'acide chroman-2-carboxylique par le chlorure de l'acide 2-phénoxybutyrique, on obtient le produit du titre.

### Caractéristiques :

Masse moléculaire pour C₂₁H₂₆N₂O₂, HCl : 374,5 g.mol⁻¹
Rendement : 39 %
Rf : 0,82 (Ethanol)
Point de fusion : 210°C

### Spectroscopie dans l'infra-rouge (KBr, νcm⁻¹) :

3005, 2850, 2810 (CH, CH₂, CH₃) ; 2500 - 2750 (NH⁺) ; 1675 (CO) ; 1610,1595 (C=C)

### Résonnance magnétique nucléaire ¹H(D.M.S.O-d₆, δ ppm) :

1,13 (t, 3H, CH₂-CH₃) ; 1,89 (q, 2H, CH₂-CH₃) ; 3,55 (m, 8H, CH₂N) ; 4,44 (s, 2H, CH₂-Ar) ; 5,15 (t, 1H, CH-C₂H₅) ; 6,94 - 7,74 (m, 10H, Ar) ; 12,04 (s, large, 1H, NH⁺).

### EXEMPLE 9 : 1-(2-PHENOXYPROPYL)-4-BENZYLPIPERAZINE (Chlorhydrate et base)

En procédant comme dans l'exemple 1 mais en remplaçant la 1-[(5-fluoro-2,3-dihydro benzofur-2-yl)carbonyl]-4-benzylpipérazine par le chlorhydrate de la 1-(2-phénoxypropionyl)-4-benzylpipérazine obtenu dans l'exemple 7, on obtient le produit du titre.

### Caractéristiques :

Masse moléculaire pour C₂₀H₂₆N₂O₂ : 310 g.mol⁻¹
Rendement : 40 %
Rf : 0,42 (Acétate d'éthyle)

### Spectroscopie dans l'infra-rouge (KBr, νcm⁻¹) :

3145, 3010, 2885, 2840, 2815 (CH, CH₂, CH₃) ; 1605 (C=C)

### Résonnance magnétique nucléaire ¹H (CDCl₃, δ ppm) :

1,27 (d, 3H, CH₃-CH) ; 3,51 (m, 10H, CH₂N et 8H pipérazine) ; 3,47 (s, 2H, CH₂-Ar) ; 4,52 (q, 1H, CH-CH₃) ; 6,84 - 7,30 (m, 10H, aromatiques).

### Chlorhydrate:

### Caractéristiques :

Masse moléculaire pour C₂₀H₂₆N₂O₂, 2HCl : 383 g.mol⁻¹
Rendement : 77 %
Rf : 0,91 (Acétate d'éthyle)
Point de fusion : 203°C

### Spectroscopie dans l'infra-rouge (KBr, ν cm⁻¹):

3095, 3020, 3000, 2975 (CH, CH₂, CH₃) ; 2650 - 2200 (NH+) ; 1605 (C=C)

### Résonnance magnétique nucléaire ¹H (D.M.S.O-d₆, δ ppm):

1,23 (d, 3H, CH₃-CH) ; 3,51 (m, 10H, CH₂N et 8H pipérazine) ; 4,36 (s, 2H, CH₂-Ar) ; 5,03 (q, 1H, CH-CH₃) ; 6,95 - 7,64 (m, 10H, aromatiques).

### EXEMPLE 10 1-(2-PHENOXYBUTYL)-4-BENZYLPIPERAZINE (Base et chlorhydrate)

En procédant comme dans l'exemple 1 mais en remplaçant la 1-[(5-fluoro-2,3-dihydro benzofur-2-yl)carbonyl]-4-benzylpipérazine par le chlorhydrate de la 1-(2-phénoxybutyryl)-4-benzyl pipérazine obtenu dans l'exemple 8, on obtient le produit du titre.

### Caractéristiques :

Masse moléculaire pour C₂₁H₂₈N₂O₂ : 324 g.mol⁻¹
Rf : 0,56 (Acétate d'éthyle)

### Spectroscopie dans l'infra-rouge (KBr, ν cm⁻¹):

3150, 3125, 2975, 2810, 2815 (CH, CH₂, CH₃) ; 1600 (C=C)

### Résonnance magnétique nucléaire ¹H(CDCl₃, δ ppm):

0,94 (t, 3H, CH₂-CH₃) ; 1,70 (q, 2H, CH₂-CH₃) ; 2,43 - 2,70 (m, 10H, CH₂-N + 8H pipérazine) ; 3,46 (s, 2H, CH₂-Ar) ; 4,26 - 4,37 (m, 1H, CH-CH₂-N) ; 6,84 - 7,30 (m, 10H aromatiques).

### Chlorhydrate:

Masse moléculaire pour C₂₁H₂₈N₂O_{2,}HCl : 397 g.mol⁻¹
Rendement : 88 %
Rf : 0,81 (Ethanol)
Point de fusion : 229°C

### Spectroscopie dans l'infra-rouge (KBr, νcm⁻¹) :

3095, 3020, 3000, 2975 (CH, CH₂, CH₃) ; 2650 - 2200 (NH⁺) ; 1605 (C=C)

### Résonnance magnétique nucléaire ¹H(CDCl₃, δppm) :

0,92(t, 3H, CH₂-CH₃) ; 1,64 (q, 2H, CH₂-CH₃) ; 3,45 (m, 10H, CH₂-N + 8H pipérazine) ; 4,32 (s, 2H, CH₂-Ar) 4,87 (m, 1H, CH-CH₂-N) ; 6,94 - 7,59 (m, 10H, aromatiques) ; 11,70 (s, large, 2H, 2NH⁺).

### EXEMPLE 11 : 1-[(7-METHOXY-2-0XO-CHROM-3-EN-4-YL)METHYL]-4-(4-METHOXYBENZYL) PIPERAZINE

Dans un erlenmeyer contenant 100 ml de tétrahydrofurane, on introduit 0,01 mole de 1-(4-méthoxybenzyl)pipérazine et 0,01 mole de 4-bromométhyl-7-méthoxy-2-oxo-chrom-3-ène. Le mélange est agité à température ambiante pendant 3 heures. On rajoute alors 0,01 mole de carbonate de potassium et on porte à reflux. La réaction est suivie par chromatographie sur couche mince. Lorsqu'elle est terminée la solution est filtrée et évaporée sous pression réduite. Le résidu est alors purifié par chromatographie sur colonne de gel de silice avec du chlorure de méthylène puis de l'acétate d'éthyle comme éluants. On obtient ainsi le produit sous forme de base.

### Caractéristiques :

Rendement : 45 %
Point de fusion : 108°C

### Spectroscopie dans l'infra-rouge (KBr νcm⁻¹) :

3100 - 2980 (CH) ; 2850 - 2800 (CH₂, CH₃) ; 1715 (COO) ; 1610 (C=C)

### Résonnance magnétique nucléaire ¹H(CDCl₃, δppm) :

2,50 (m, 8H, CH₂N) ; 3,45 (s, 2H C₆H₄-CH₂) ; 3,52 (s, 2H,Ar-CH₂ ; 3,79 (s, 3H OCH₃) ; 3,85 (s, 3H OCH₃) ; 6,15 (s, 1H, H₃) ; 6,85 (m, 4H, α des OCH₃) ; 7,20 (α, 2H, β des OCH₃, J = 8Hz) ; 7,70 (α, 1H, H₅, J = 8Hz)

### Obtention du chlorhydrate

La base (0,002 mole) est dissoute dans 10 ml d'isopropanol chaud. Après dissolution, on verse 5 ml d'isopropanol chlorhydrique. Après refroidissement le produit est filtré et séché.

### Caractéristiques :

Rendement : 83 %
Point de fusion : 208°C

### EXEMPLE 12: 1-(2-PHENOXY-2-METHYLPROPIONYL)-4-BENZYL-PIPERAZINE

Dans un erlenmeyer contenant une solution de 0,01 mole de 1-benzylpipérazine dans 100 ml de chlorure de méthylène, on introduit une solution de 0,01 mole de chlorure de l'acide de 2-phénoxyisobutyrique (obtenue par action de chlorure de thionyle sur l'acide 2-phénoxy isobutyrique) dans 100 ml de chlorure de méthylène. Le mélange est agité pendant 5 heures à température ambiante. Filtrer. Evaporer à sec. Reprendre par l'eau. Alcaliniser. Extraire au chloroforme. Réunir les phases organiques. Sécher et évaporer.

### EXEMPLE 13: 1-(2-PHENOXY 2-METHYLPROPYL)-4-BENZYL-PIPERAZINE

A 0,01 mole du produit obtenu dans l'exemple 12 précédent dissous dans 100 ml de tétrahydrofuranne, on ajoute 1 g d'hydrure de lithium aluminium. L'opération est répétée trois heures plus tard. Après la fin de la réaction, suivie par chromatographie couche mince, l'excés d'hydrure est détruit par 20 ml de méthanol. Après évaporation à sec, le résidu est repris par 5 ml d'eau et par 100 ml d'acétate d'éthyle. La phase organique est filtrée, évaporée et purifiée par chromatographie sur colonne de gel de silice en utilisant le chlorure de méthylène et l'acétate d'éthyle comme éluant. On obtient ainsi le produit sous forme de base.

### Caractéristiques :

Rendement : 33 %

### Spectroscopie dans l'infra-rouge (KBr, νcm⁻¹) :

3100 - 3000 (CH) ; 2970 - 2810 (CH₂-CH₃) ; 1591 (C=C)

### Résonnance magnétique nucléaire ¹H(CDCl₃, δppm) :

1,26 (singulet 6H, 2CH₃) ; 3,50 (singulet 2H Ar-CH₂-N)

### Obtention du chlorhydrate

Dissoudre 0,01 mole de base obtenue dans 10 ml d'isopropanol chaud. Après dissolution, verser 5 ml d'isopropanol chlorhydrique. Après refroidissement, filtrer et sécher.

### Caractéristiques :

Rendement : 86 %
Point de fusion : 189°C

### EXEMPLE 14 : 1-[2-(4-CHLOROPHENOXY)PROPIONYL]-4-BENZYLPIPERAZINE

En procédant comme dans l'exemple 12 mais en utilisant le chlorure de l'acide 2-(4-chlorophénoxy)propionique, on obtient le produit du titre.

### EXEMPLE 15 : 1-[2-(4-CHLOROPHENOXY)PROPYL]-4-BENZYLPIPERAZINE

En procédant comme dans l'exemple 13 mais en utilisant la 1-[2-(4-chlorophénoxy)propionyl]-4-benzylpipérazine obtenue dans l'exemple 14, on obtient le produit du titre.

### EXEMPLE 16 : 1-[2-(4-CHLOROPHENOXY)-2-METHYLPROPIONYL]-4-BENZYL PIPERAZINE

En procédant comme dans l'exemple 12 (stade A) mais en utilisant le chlorure de l'acide 2-(4-chlorophénoxy)-2-méthyl propionique, on obtient le produit du titre.

### EXEMPLE 17 : 1-[2-(4-CHLOROPHENOXY)-2-METHYLPROPYL]-4-BENZYLPIPERAZINE

En procédant comme dans l'exemple 13 mais en utilisant la 1-[2-(4-chlorophénoxy)-2-méthylpropionyl]-4-benzylpipérazine obtenue à l'exemple 16, on obtient le produit du titre.

### EXEMPLE 18 : 1-(2-PHENOXY PROPIONYL)-4-(4-METHOXYBENZYL)PIPERAZINE

En procédant comme dans l'exemple 12 (stade A) mais en utilisant le chlorure de l'acide 2-phénoxy propionique et la 1-(4-méthoxybenzyl)pipérazine, on obtient le produit du titre.

### EXEMPLE 19 : 1-(2-PHENOXYPROPYL)-4-(4-METHOXYBENZYL)PIPERAZINE

En procédant comme dans l'exemple 13 mais en utilisant la 1-(2-phénoxypropionyl)-4-(4-méthoxybenzyl)pipérazine obtenue à l'exemple 18, on obtient le produit du titre.

### EXEMPLE 20 : 1-(2-PHENOXY-2 -METHYLPROPIONYL)-4-(4-METHOXYBENZYL) PIPERAZINE

En procédant comme dans l'exemple 12 mais en utilisant le chlorure de l'acide 2-phénoxy-2-méthylpropionique, et la 1-(4-méthoxybenzyl)pipérazine, on obtient le produit du titre.

### EXEMPLE 21 : 1-(2-PHENOXY-2-METHYLPROPYL)-4-(4-METHOXYBENZYL) PIPERAZINE

En procédant comme dans l'exemple 13 mais en utilisant la 1-(2-phénoxy-2-méthyl propionyl)-4-(4-méthoxy benzyl)pipérazine, obtenue dans l'exemple 20, on obtient le produit du titre.

### EXEMPLES 22 A 55 :

En procédant comme dans l'exemple 2 (stade A) mais en remplaçant le chlorure de l'acide chroman-2-carboxylique par le chlorure d'acide approprié, on obtient les produits des exemples suivants :

### EXEMPLE 22 : 1-[2-(3-FLUOROPHENOXY)PROPIONYL]-4-BENZYLPIPERAZINE

### EXEMPLE 23 : 1-[2-(2,4-DIETHYLPHENOXY)PROPIONYL]-4-BENZYLPIPERAZINE

### EXEMPLE 24 : 1-[2-(2-METHYL-4-lSOPROPYLPHENOXY)PROPIONYL]-4-BENZYL PIPERAZINE

### EXEMPLE 25 : 1-[2-(4-TRIFLUOROMETHYLPHENOXY)PROPIONYL]-4-BENZYL PIPERAZINE

### EXEMPLE 26 : 1-[2-(3-ETHYLPHENOXY)PROPIONYL]-4-BENZYLPIPERAZINE

### EXEMPLE 27 : 1-[2-(4-ETHYLPHENOXY)PROPIONYL]-4-BENZYLPIPERAZINE

### EXEMPLE 28 : 1-[2-(4-ETHOXYPHENOXY)PROPIONYL]-4-BENZYLPIPERAZINE

### EXEMPLE 29 : 1-[2-(4-PROPOXYPHENOXY)PROPIONYL]-4-BENZYLPIPERAZINE

### EXEMPLE 30 : 1-[2-(2-METHYL-4-SEC-BUTYLPHENOXY)PROPIONYL]-4-BENZYL PIPERAZINE

### EXEMPLE 31 : 1-[2-(4-ISOPROPYLPHENOXY)PROPIONYL]-4-BENZYLPIPERAZINE

### EXEMPLE 32 : 1-[2-(3-BROMOPHENOXY)-2-METHYLPROPIONYL]-4-BENZYL PIPERAZINE

### EXEMPLE 33 : 1-[2-(4-ETHOXYPHENOXY)-2-METHYLPROPIONYL]-4-BENZYL PIPERAZINE

### EXEMPLE 34 : 1-[2-(2,4-DICHLOROPHENOXY)-2-METHYLPROPIONYL]-4-BENZYL PIPERAZINE

### EXEMPLE 35 : 1-[2-(3-TRIFLUOROMETHYLPHENOXY)BUTYRYL]-4-BENZYL PIPERAZINE

### EXEMPLE 36 : 1-[2-(4-TERTIOBUTYLPHENOXY)BUTYRYL]-4-BENZYLPIPERAZINE

### EXEMPLE 37 : 1-[2-(3-ISOPROPYLPHENOXY)BUTYRYL]-4-BENZYLPIPERAZINE

### EXEMPLE 38 : 1-[2-(4-FLUOROPHENOXY)BUTYRYL]-4-BENZYLPIPERAZINE

### EXEMPLE 39 : 1-[2-(3-IODOPHENOXY)BUTYRYL]-4-BENZYLPIPERAZINE

### EXEMPLE 40 : 1-[2-(2,4-DIIODOPHENOXY)BUTYRYL]-4-BENZYLPIPERAZINE

### EXEMPLE 41 : 1-[2-(2,4-DICHLOROPHENOXY)BUTYRYL]-4-BENZYLPIPERAZINE

### EXEMPLE 42 : 1-[2-(4-BROMOPHENOXY)BUTYRYL]-4-BENZYLPIPERAZINE

### EXEMPLE 43 : 1-[2-(4-METHYLPHENOXY)BUTYRYL]-4-BENZYLPIPERAZINE

### EXEMPLE 44 : 1-[2-(2-TRIFLUOROMETHYL-4-FLUOROPHENOXY)BUTYRYL]-4-BENZYLPIPERAZINE

### EXEMPLE 45 : 1-[2-(3-CHLOROPHENOXY)-2-METHYLBUTYRYL]-4-BENZYL PIPERAZINE

### EXEMPLE 46 : 1-(2-PHENOXYPENTANOYL)-4-BENZYLPIPERAZINE

### EXEMPLE 47 : 1-(2-PHENOXY-2-METHYLPENTANOYL)-4-BENZYLPIPERAZINE

### EXEMPLE 48 : 1-[2-(4-CHLOROPHENOXY)PENTANOYL]-4-BENZYLPIPERAZINE

### EXEMPLE 49 : 1-[2-(4-CHLOROPHENOXY)-2-METHYLPENTANOYL]-4-BENZYL PIPERAZINE

### EXEMPLE 50 : 1-[2-(4-HEXYLPHENOXY)PENTANOYL]-4-BENZYLPIPERAZINE

### EXEMPLE 51 : 1-[2-(3-CHLOROPHENOXY)PENTANOYL]-4-BENZYLPIPERAZINE

### EXEMPLE 52 : 1-(2-PHENOXYHEXANOYL)-4-BENZYLPIPERAZINE

### EXEMPLE 53 : 1-[2-(3,4-DICHLOROPHENOXY)HEXANOYL]-4-BENZYLPIPERAZINE

### EXEMPLE 54 : 1-[2-(4-METHOXYPHENOXY)HEPTANOYL]-4-BENZYLPIPERAZINE

### EXEMPLE 55 : 1-(2-PHENOXYOCTANOYL)-4-BENZYLPIPERAZINE

### EXEMPLES 56 A 89 :

En réduisant comme dans l'exemple 1 mais en utilisant au départ les composés des exemples 22 à 55, on obtient respectivement les produits suivants :

### EXEMPLE 56 : 1-[2-(3-FLUOROPHENOXY)PROPYL]-4-BENZYLPIPERAZINE

### EXEMPLE 57 : 1-[2-(2,4-DIETHYLPHENOXY)PROPYL]-4-BENZYLPIPERAZINE

### EXEMPLE 58 : 1-[2-(2-METHYL-4-ISOPROPYLPHENOXY)PROPYL]-4-BENZYL PIPERAZINE

### EXEMPLE 59 : 1-[2-(4-TRIFLUOROMETHYLPHENOXY)PROPYL]-4-BENZYL PIPERAZINE

### EXEMPLE 60 : 1-[2-(3-ETHYLPHENOXY)PROPYL]-4-BENZYLPIPERAZINE

### EXEMPLE 61 : 1-[2-(4-ETHYLPHENOXY)PROPYL]-4-BENZYLPIPERAZINE

### EXEMPLE 62 : 1-[2-(4-ETHOXYPHENOXY)PROPYL]-4-BENZYLPIPERAZINE

### EXEMPLE 63 : 1-[2-(4-PROPOXYPHENOXY)PROPYL]-4-BENZYLPIPERAZINE

### EXEMPLE 64 : 1-[2-(2-METHYL-4-SEC-BUTYLPHENOXY)PROPYL]-4-BENZYL PIPERAZINE

### EXEMPLE 65 : 1-[2-(4-ISOPROPYLPHENOXY)PROPYL]-4-BENZYLPIPERAZINE

### EXEMPLE 66 : 1-[2-(3-BROMOPHENOXY)-2-METHYLPROPYL]-4-BENZYLPIPERAZINE

### EXEMPLE 67 : 1-[2-(4-ETHOXYPHENOXY)-2-METHYLPROPYL]-4-BENZYLPIPERAZINE

### EXEMPLE 68 : 1-[2-(2,4-DICHLOROPHENOXY)-2-METHYLPROPYL]-4-BENZYL PIPERAZINE

### EXEMPLE 69 : 1-[2-(3-TRIFLUOROMETHYLPHENOXY)BUTYL]-4-BENZYLPIPERAZINE

### EXEMPLE 70 : 1-[2-(4-TERTIOBUTYLPHENOXY)BUTYL]-4-BENZYLPIPERAZINE

### EXEMPLE 71 : 1-[2-(3-ISOPROPYLPHENOXY)BUTYL]-4-BENZYLPIPERAZINE

### EXEMPLE 72 : 1-[2-(4-FLUOROPHENOXY)BUTYL]-4-BENZYLPIPERAZINE

### EXEMPLE 73 : 1-[2-(3-IODOPHENOXY)BUTYL]-4-BENZYLPIPERAZINE

### EXEMPLE 74 : 1-[2-(2,4-DIIODOPHENOXY)BUTYL]-4-BENZYLPIPERAZINE

### EXEMPLE 75 : 1-[2-(2,4-DICHLOROPHENOXY)BUTYL]-4-BENZYLPIPERAZINE

### EXEMPLE 76 : 1-[2-(4-BROMOPHENOXY)BUTYL]-4-BENZYLPIPERAZINE

### EXEMPLE 77: 1-[2-(4-METHYLPHENOXY)BUTYL]-4-BENZYLPIPERAZINE

### EXEMPLE 78 : 1-[2-(2-TRIFLUOROMETHYL-4-FLUOROPHENOXY)BUTYL]-4-BENZYL PIPERAZINE

### EXEMPLE 79 : 1-[2-(3-CHLOROPHENOXY)-2-METHYL-BUTYL]-4-BENZYLPIPERAZINE

### EXEMPLE 80 : 1-(2-PHENOXYPENTYL)-4BENZYLPIPERAZINE

### EXEMPLE 81 : 1-(2-PHENOXY-2-METHYLPENTYL)-4-BENZYLPIPERAZINE

### EXEMPLE 82 : 1-[2-(4-CHLOROPHENOXY)PENTYL]-4-BENZYLPIPERAZINE

### EXEMPLE 83 : 1-[2-(4-CHLOROPHENOXY)-2-METHYLPENTYL]-4-BENZYLPIPERAZINE

### EXEMPLE 84 : 1-[2-(4-HEXYLPHENOXY)PENTYL]-4-BENZYLPIPERAZINE

### EXEMPLE 85 : 1-[2-(3-CHLOROPHENOXY)PENTYL]-4-BENZYLPIPERAZINE

### EXEMPLE 86 : 1-(2-PHENOXYHEXYL)-4-BENZYLPIPERAZINE

### EXEMPLE 87 : 1-[2-(3,4-DICHLOROPHENOXY)HEXYL]-4-BENZYLPIPERAZINE

### EXEMPLE 88 : 1-[2-(4-METHOXYPHENOXY)HEPTYL]-4-BENZYLPIPERAZINE

### EXEMPLE 89 : 1-(2-PHENOXYOCTYL)-4-BENZYLPIPERAZINE

### EXEMPLE 90 : 1-[(5-FLUOROCHROMAN-2-YL)METHYL]-4-BENZYLPIPERAZINE

En procédant comme dans l'exemple 1 mais en utilisant au départ la 1-[(5-fluoro chroman-2-yl)carbonyl]-4-benzylpipérazine résultant de la condensation de l'acide 5-fluoro chroman-2-carboxylique avec la N-benzyl pipérazine, on obtient le composé du titre.

### EXEMPLE 91 : 1-[(CHROM-3-EN-2-YL)METHYL]-4-BENZYLPIPERAZINE

En procédant comme dans l'exemple 1 mais en utilisant au départ la 1-[(chrom-3-èn-2-yl) carbonyl]4-benzylpipérazine résultant de la condensation de l'acide chrom-3-èn-2-carboxylique avec la N-benzyl pipérazine, on obtient le composé du titre.

### EXEMPLES 92 A 106:

En procédant comme dans l'exemple 2 mais en remplaçant la 1-benzylpipérazine par la pipérazine appropriée, on obtient les produits suivants:

### EXEMPLE 92 : 1-[(CHROMAN-2-YL)METHYL]-4-(4-METHOXYBENZYL)PIPERAZINE

### EXEMPLE 93 : 1-[(CHROMAN-2-YL)METHYL]-4-(2-METHOXYBENZYL)PIPERAZINE

### EXEMPLE 94 : 1-[(CHROMAN-2-YL)METHYL]-4-(3-METHOXYBENZYL)PIPERAZINE

### EXEMPLE 95 : 1-[(CHROMAN-2-YL)METHYL]-4-(4-HYDROXYBENZYL)PIPERAZINE

### EXEMPLE 96 : 1-[(CHROMAN-2-YL)METHYL]-4-(4-ISOPROPOXYBENZYL)PIPERAZINE

### EXEMPLE 97 : 1-[(CHROMAN-2-YL)METHYL]-4-(4-TRIFLUOROMETHYLBENZYL) PIPERAZINE

### EXEMPLE 98 : 1-[(CHROMAN-2-YL)METHYL]-4-(4-METHYLBENZYL)PIPERAZINE

### EXEMPLE 99 : 1-[(CHROMAN-2-YL)METHYL]-4-(4-CHLOROBENZYL)PIPERAZINE

### EXEMPLE 100 : 1-[(CHROMAN-2-YL)METHYL]-4-(4-FLUOROBENZYL)PIPERAZINE

### EXEMPLE 101 : 1-[(CHROMAN-2-YL)METHYL]-4-(3,4-DICHLOROBENZYL)PIPERAZINE

### EXEMPLE 102 : 1-[(CHROMAN-2-YL)METHYL]-4-(2,4-DICHLOROBENZYL)PIPERAZINE

### EXEMPLE 103 : 1-[(CHROMAN-2-YL)METHYL]-4-(2,3,4-TRIMETHOXYBENZYL) PIPERAZINE

### EXEMPLE 104 : 1-[(CHROMAN-2-YL)METHYL]-4-(NAPHTYLMETHYL)PIPERAZINE

### EXEMPLE 105 : 1-[(CHROMAN-2-YL)METHYL)-4-(PHENYLETHYL)PIPERAZINE

### EXEMPLE 106 : 1-[(CHROMAN-2-YL)METHYL]-4-(PHENYLBUTYL)PIPERAZINE

### ETUDE PHARMACOLOGIQUE DES DERIVES DE L'INVENTION

### EXEMPLE A : ETUDE DE LA TOXICITE AIGUE

La toxicité aigue a été appréciée après administration orale à des lots de 8 souris (26 ± 2 grammes) d'une dose de 100 mg.kg⁻¹ des composés de l'invention. Les animaux ont été observés à intervalles réguliers au cours de la première journée quotidiennement pendant les deux semaines suivant le traitement.
Il apparaît que les composés de l'invention sont totalement atoxiques. Ils n'entrainent aucun décès après administration à une dose de 100 mg.kg⁻¹ et on ne constate pas de troubles après administration de cette dose.

### EXEMPLE B : BILAN RECEPTORIEL D'AFFINITE IN VITRO

Les produits sont testés sur chaque récepteur à 5 concentrations différentes (10⁻⁵M, 10⁻⁶M, 10⁻⁷M, 10⁻⁸M, 10⁻⁹M) en triplicata. Lorsque le coefficient de liaison IC₅₀ (concentration de produit qui déplace 50 % du radioligand) est inférieur à une concentration de 10⁻⁶M, le Ki est mesuré en utilisant 12 concentrations du produit.
Le tableau A regroupe les récepteurs pour lesquels l'affinité des composés de l'invention a été déterminée, le tissu choisi, la concentration retenue pour déterminer la fraction non spécifique et le radioligand utilisé pour marquer le récepteur.

**Tableau A :**

| Bilan réceptoriel d'affinité in vitro : | | | |
|---|---|---|---|
| **Récepteur ou site** | **Radioligand** | **Fraction non spécifique** | **Tissu** |
| 5-HT_{1A} | 8-OH DPAT | 10⁻⁵M Buspirone | Hippocampe |
| 5-HT_{1B} | [³H] Cyanopindolol | 10⁻⁵M Sérotonine froide | Cerveau de rat |
| 5-HT_{1C} | N-méthyl Mésulergine | 10⁻⁵M Miansérine | Cortex frontal Hippocampe |
| 5-HT₂ | [³H] Kétansérine | 10⁻⁵M Spipérone | Cortex frontal |
| 5-HT₃ | [³H] Quipazine | 10⁻⁵M Zacopride | lléon de rat |
| α¹ | [³H] Prazosine | 10⁻⁵M Phentolamine | Cerveau de rat |
| α₂ | [³H] Rauwolscine | 10⁻⁵M Yohimbine | Cerveau de rat |
| D₁ | [³H] SCH 23390 | 10⁻⁶M Butaclamol | Striatum de rat |
| D₂ | [³H] Raclopride | 10⁻⁶M Halopéridol | Striatum de rat |
| M₁ | [³H] Télenzépine | 10⁻⁵M Atropine | Cortex |
| H₁ | [³H] Pyrilamine | 10⁻⁶M Chlorphéniramine | Cortex de veau |
| σ | [³H]DTG ou [³H] 3-PPP | 10⁻⁶M 3-PPP | Hippocampe de veau |
| σ₁ | [³H]pentazocine | 5.10⁻⁶M Halopéridol | Cerveau de cobaye |
| σ₂ | [³H] DTG | 5.10⁻⁶M Halopéridol | Cerveau de cobaye |

Les résultats du test ont montré que les composés de l'invention sont de puissants et sélectifs ligands des récepteurs σ. Des exemples de liaison aux récepteurs σ sont donnés dans le tableau B ci-dessous.

**Tableau B :**

| Résultats du test de liaison aux récepteurs σ : | | | |
|---|---|---|---|
| **Composé** | **Récepteur** | **Radioligand** | **IC**_{**50**}**(M)** |
| Exemple 1 | σ | [³H] DTG | 9,1.10⁻⁹ |
| Exemple 3 | σ | [³H] DTG | 1,3.10⁻⁹ |
| Exemple 4 | σ | [³H] DTG | 7.10⁻⁹ |
| Exemple 9 | σ | [³H] DTG | 4.10⁻⁹ |
| | σ₁ | [³H] pentazocine | 1,3.10⁻⁹ |
| | σ₂ | [³H] DTG | 4.10⁻⁸ |

### EXEMPLE C : ANTAGONISME DE L'HYPERMOTILITE INDUITE PAR L'AMPHETAMINE

Ce test a été mis au point par Costall B., et al (Brain Research, 1977, 123:89-111).

On injecte à des groupes de 10 souris NMRI-CERJ par voie intrapéritonéale (IP) 4 mg.kg⁻¹ de d-amphétamine juste après le composé à tester (injecté aussi par voie IP), et on place les souris dans un actimètre pendant 30 minutes.
Le nombre d'interruptions de cellules photoélectriques est compté, de même que le comportement stéréotypé.

L'activité des composés testés est exprimée en pourcentage de l'antagonisme de l'hyperactivité induite par l'amphétamine.

Les résultats du test montrent que les composés de l'invention sont de puissants antagonistes de l'hypermotilité induite par l'amphétamine. Ainsi par exemple les composés des exemples 1 et 4, à une dose de 64 mg/kg, antagonisent respectivement à 43 % et à 61 % l'hypermotilité induite par l'amphétamine. Les produits de l'invention sont ainsi de bons candidats pour le traitement des troubles du système nerveux central.

### EXEMPLE D : ANTAGONISME DE L'HYPERACTIVITE INDUITE PAR LA N-ALLYL NORMETAZOCINE

Ce test a été mis au point par Snyder S.H. et al (J. Neuropsychiatry, 1989, 1: 7-15).

La N-allylnormetazocine ((+) SKF 10047) induit un comportement psychotique chez l'homme et est le prototype d'agoniste pour les récepteurs σ.

La mesure de l'hyperactivité induite par ce produit est donc utilisée comme modèle alternatif pour détecter l'activité antipsychotique des composés agissant sur les récepteurs σ.

Un groupe de 12 rats est traité avec le composé à tester avant l'administration sous-cutanée de 50 mg/kg de la N-allylnormetazocine. 30 minutes plus tard, les animaux sont placés dans un actimètre pendant 30 minutes.

L'haloperidol est utilisé comme composé de référence.

Les résultats du test montrent que les composés de l'invention sont de puissants antagonistes de l'hyperactivité induite par la N-allylnormétazocine. Ainsi par exemple le composé de l'exemple 9 à une dose de 32 mg.kg⁻¹ antagonise de 42 % l'hypermotilité induite par la N-allylnormétazocine.

Ce test confirme donc l'intérêt des produits de l'invention pour le traitement des troubles du système nerveux central.

### EXEMPLE E : RECHERCHE D'EFFET CATALEPSIGENE

Ce test a été mis au point par Chermat R. et al (J. Pharmacol., 1975, 6: 493-496).

6 groupes de rats Wistar ont reçu une injection par voie intrapéritonéale des composés de l'invention. Une activité catalepsigène à 30 minutes d'intervalle est recherchée. La prochlorpérazine est utilisée comme référence.

Les résultats de ce test révèlent que les composés de l'invention présentent un effet catalepsigène très faible en comparaison avec la prochlorpérazine dans les mêmes conditions de test. Ce résultat confirme l'absence d'effets secondaires de type extrapyramidaux des produits de l'invention qui pouvait être attendu suite aux résultats des tests de liaison (exemple B).

### EXEMPLE F : ETUDE DE L'ACTIVITE ANTIDEPRESSIVE DES COMPOSES DE L'INVENTION

### PRINCIPE :

L'étude des produits est réalisée sur le modèle du "renoncement appris" qui consiste à induire chez l'animal, par une série d'évènements aversifs incontrôlables, un déficit lors des tâches d'évitement ultérieures.

### PROTOCOLE :

Ce test a été mis au point par Sherman A.D., Sacquitne J.L., et Petty F. (Pharmacol. Biochem. Behav, 1982, 16 : 449-454). On utilise des rats mâles Wistar d'un poids compris entre 180 et 200 grammes. Les animaux sont maintenus à l'animalerie une semaine avant le test, dans des boîtes en plastique, par groupes de 10, à une température ambiante de 21°C ± 1°C, avec libre accès à l'eau et à la nourriture.

Les animaux sont ensuite isolés dans des boîtes de petites dimensions et sont soumis à 60 chocs électriques inévitables (0,8 mA toutes les minutes ± 15 secondes). Un groupe de rats témoins ne reçoit pas de chocs électriques.

La capacité des animaux à réaliser un apprentissage d'évitement (passage d'un compartiment à l'autre, afin d'éviter les chocs électriques) est appréciée 48 heures après et pendant 3 jours consécutifs. Lors des séances d'apprentissage, les animaux subissent 2 essais par minute pendant 15 minutes. Le nombre d'échecs d'échappement est noté pour chaque rat. Les animaux sont traités (injection de 0,5 ml/100 g par voie intrapéritonéale) à jeun 6 heures après les chocs inévitables et 4 jours de suite, le matin 30 minutes avant la séance d'apprentissage et le soir entre 18 et 19 h.

Les produits étudiés sont mis en solution dans de l'eau distillée.
Les produits étudiés sont administrés aux doses 0,05 mg/kg/jour.

### RESULTATS :

Le test prouve que certains produits de l'invention diminuent significativement le nombre d'échecs d'échappement ce qui traduit, pour certains produits de l'invention, une forte activité de type antidépressive.

### EXEMPLE G : ETUDE DE L'ACTIVITE ANXIOLYTIQUE-TEST DIT DES CAGES CLAIRE/ OBSCURE CHEZ LA SOURIS

### PROTOCOLE :

Ce test a été mis au point par Crawley et al. (Pharmacol. Biochem. Behav. 1981, 15 (5) : 695-699), puis modifié et comportementalement validé.

Il s'agit de deux cages de tailles égales (20 x 20 x 14 cm) en PVC. L'une est fortement éclairée par une lampe de 100 W (lumière "froide"), l'autre est obscurcie. Les deux cages sont séparées l'une de l'autre au moyen d'un petit tunnel opaque (5 x 7 cm). Les souris sont individuellement introduites dans la cage obscure. On enregistre, au moyen de claviers reliés à un ordinateur, durant 5 minutes, le temps passé par les animaux dans la cage éclairée ainsi que le nombre des transitions entre la cage obscure et la cage éclairée. Chaque groupe expérimental comprend au minimum 15 animaux.

### RESULTATS :

L'administration, par voie intrapéritonéale des produits de l'invention entraine une augmentation du temps passé par les souris dans la cage éclairée et du nombre de transitions entre la cage obscure et la cage éclairée.

Cette augmentation significative des deux paramètres étudiés montre la remarquable activité anxiolytique des composés de l'invention.

### EXEMPLE H : RECHERCHE D'UNE ACTIVITE DE TYPE ANTIARTHRITIQUE CHEZ LE RAT

### PROTOCOLE :

On utilise des groupes de 5 rats Lewis mâle ou femelle d'un poids de 130 g à 150 g. Une suspension de 0,3 mg de Mycobactérium tuberculosis tués dans 0,1 ml d'huile minérale (Adjuvant complet de Freund, CFA) est administrée dans la région de la patte arrière le Jour 1. Les volumes des pattes arrières sont mesurés par déplacement d'eau les Jours 0, 1, 5, 14 et 18. Les produits à tester sont placés en suspension dans la carboxyméthyl cellulose et administrés oralement pendant 5 jours consécutifs des Jours 1 à 5.

### RESULTATS :

On observe après administration des produits de l'invention une diminution significative du volume des pattes arrières dans la phase précoce et dans la phase tardive de l'inflammation (après J 14).Les produits de l'invention sont donc de bons candidats dans le traitement de l'arthrite.

### EXEMPLE I : COMPOSITION PHARMACEUTIQUE

1. Comprimés dosés à 0,1 mg de 1-[(5-méthoxychroman-3-yl)méthyl]-4-benzyl pipérazine utilisables dans le traitement des troubles du système nerveux central.
   Formule pour 10 000 comprimés :
   - 1-[(5-méthoxychroman-3-yl)méthyl]-4-benzylpipérazine 1 g
   - Amidon de blé 75 g
   - Amidon de maïs 75 g
   - Lactose 325 g
   - Stéarate de magnésium 10 g
   - Silice 5 g
   - Hydroxypropyl cellulose 10 g
2. Comprimés dosés à 50 mg de 1-[(7-méthoxy-2-oxo-chrom-3-èn-4-yl)méthyl]-4-(4-méthoxybenzyl)pipérazine utilisables dans le traitement de l'arthrite chronique.
   Formule pour 1000 comprimés
   - 1-[(7-méthoxy-2-oxo-chrom-3-èn-4-yl)méthyl]-4-(4-méthoxybenzyl)pipérazine 50 g
   - Amidon de blé 150 g
   - Amidon de maïs 150 g
   - Lactose 450 g
   - Stéarate de magnésium 10 g
   - Silice 5 g
   - Hydroxypropyl cellulose 10 g

## Revendications

1. Composé de formule (I) : 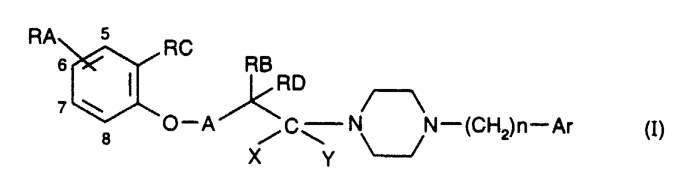 dans laquelle
- Ar représente un groupe phényle, naphtyle, phényle substitué ou naphtyle substitué,
- n représente un entier compris inclusivement entre 1 et 4,
. RB représente un groupement alkyle, et dans ce cas A représente une liaison simple et RA et RC, identiques ou différents, représentent indépendamment l'un de l'autre un atome d'hydrogène ou un groupement choisi parmi halogène, alkyle, alkyle substitué par un ou plusieurs halogènes, et alkoxy,
. ou RB et RC forment ensemble un pont -(CH₂)_{q}- avec q valant 0, 1 ou 2 et dans ce cas A représente un pont -(CH₂)ₚ- avec p représentant 0, 1 ou 2 et tel que p + q = 1 ou 2, et dans ce cas RA représente un groupement hydroxy ou alkoxy situé en position 5 du noyau aromatique qui le porte ou RA représente un atome d'hydrogène ou d'halogène, en position quelconque du cycle aromatique,
. ou RB et RC forment ensemble un pont -CH=, et la liaison qui le lie au cycle aromatique est simple, et dans ce cas A représente un groupement CH₂ et RA représente un atome d'hydrogène, un groupement hydroxy ou alkoxy situé en position 5 du cycle aromatique qui le porte,
. ou RB et RC forment ensemble une liaison simple et alors A représente un groupement 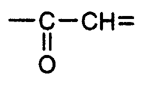 le carbonyle étant lié à l'oxygène et la liaison joignant A au carbone porteur de la chaîne latérale est double, et dans ce cas RA représente un atome d'hydrogène ou un groupement hydroxy ou alkoxy,
- lorsque RB représente un groupement alkyle, X et Y représentent chacun deux atomes d'hydrogène ou forment ensemble avec l'atome de carbone qui les porte un groupement C=O, et RD représente un atome d'hydrogène ou un groupement alkyle,
- lorsque RB et RC forment un pont, X et Y représentent chacun deux atomes d'hydrogène et RD, qui n'existe que lorsque toutes les liaisons du carbone qui le porte sont simples, représente un atome d'hydrogène,
étant entendu que, sauf mention contraire,
- les termes "alkyle" et "alkoxy" désignent des groupements saturés linéaires ou ramifiés contenant de 1 à 6 atomes de carbone,
- le terme "substitué" affectant les substituants phényle et naphtyle signifie que ceux-ci peuvent être substitués par un à trois groupements choisis parmi hydroxy, alkyle, alkyle substitué par un ou plusieurs halogènes, alkoxy, et halogène,
leurs isomères optiques sous forme pure ou sous forme de mélange, et leurs sels d'addition à un acide ou une base pharmaceutiquement acceptable.

2. Composé de formule (I) selon la revendication 1 qui est la 1-[(5-fluoro-2,3-dihydrobenzofur-2-yl)méthyl]-4-benzylpipérazine.

3. Composé de formule (I) selon la revendication 1 qui est la 1-[(2,3-dihydrobenzofur-2-yl)méthyl]-4-benzylpipérazine.

4. Composé de formule (I) selon la revendication 1 qui est la 1-[(chroman-2-yl)méthyl)]-4-benzylpipérazine.

5. Composé de formule (I) selon la revendication 1 qui est la 1-[(7-méthoxy-[2H]-2-oxochrom-3-èn-4-yl)méthyl]-4-benzylpipérazine.

6. Composé de formule (I) selon la revendication 1 qui est la 1-[(7-méthoxy-[2H]-2-oxochrom-3-èn-4-yl)méthyl]-4-(4-méthoxybenzyl)pipérazine.

7. Composé de formule (I) selon la revendication 1 qui est la 1-[2-(phénoxy)-propyl]-4-benzylpipérazine.

8. Procédé de préparation des composés de formule (I), selon la revendication 1, caractérisé en ce que l'on fait réagir un composé de formule (II) : 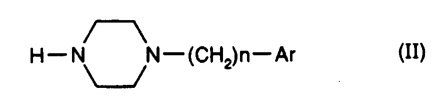 dans laquelle Ar et n sont tels que définis dans la revendication 1,
- soit avec un composé de formule (III) : 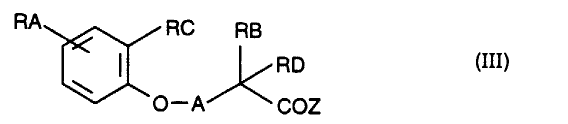 dans laquelle RA, RB, RC, RD et A sont tels que définis dans la revendication 1, Z représentant un groupement hydroxy ou un atome de chlore,
pour obtenir un composé de formule (Ia) : 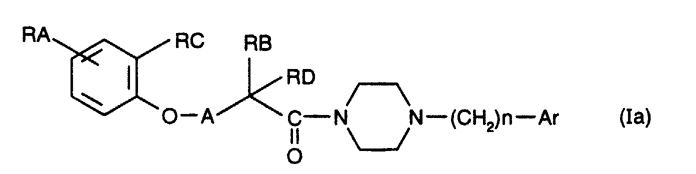 dans laquelle RA, RB, RC, RD, Ar, n et A sont tels que définis précédemment, lequel composé (Ia) peut être soumis, en fonction de la structure du composé de formule (I) que l'on souhaite obtenir, à une réduction pour donner un composé de formule(Ib) : 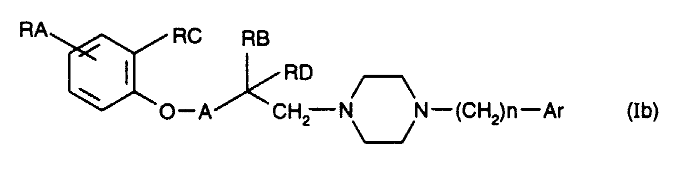 où RA, RB, RC, RD, Ar, n et A ont la même définition que précédemment,
- soit avec un composé de formule (IV) en présence d'un agent alcalin : 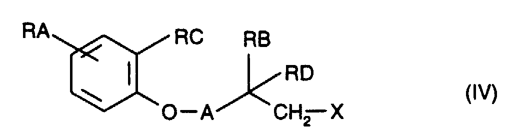 dans laquelle RA, RB, RC, RD et A sont tels que définis précédemment et X représente un atome d'halogène, pour obtenir un composé de formule (Ib) telle que définie précédemment, les composés de formule (Ia) et (Ib) formant l'ensemble des composés de formule (I), selon la revendication 1, pouvant être séparés en leurs différents isomères optiques sous forme pure ou sous forme de mélange et salifiés avec un acide ou une base pharmaceutiquement acceptable.

9. Compositions pharmaceutiques contenant comme principe actif au moins un composé selon l'une des revendications 1 à 7 en combinaison avec un ou plusieurs excipients pharmaceutiquement acceptables.

10. Compositions pharmaceutiques selon la revendication 9 utiles dans la prévention ou le traitement des troubles du système nerveux central ainsi que dans les maladies inflammatoires d'origine immunitaire.

## Claims

1. Compound of formula (I): 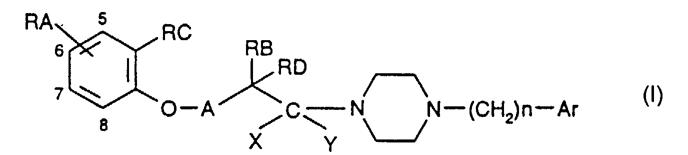 in which
- Ar represents a phenyl, naphthyl, substituted phenyl or substituted naphthyl group,
- n represents an integer from 1 to 4 inclusive,
. RB represents an alkyl group, and in that case A represents a single bond and RA and RC, which may be identical or different, each independently of the other represents a hydrogen atom or a group selected from halogen, alkyl, alkyl substituted by one or more halogen atoms, and alkoxy,
. or RB and RC together form a -(CH₂)_{q}- bridge wherein q is 0, 1 or 2, and in that case A represents a -(CH₂)ₚ- bridge wherein p represents 0, 1 or 2, such that p + q = 1 or 2, and in that case RA represents a hydroxy or alkoxy group located in the 5-position of the aromatic ring carrying it or RA represents a hydrogen or halogen atom in any position of the aromatic ring,
. or RB and RC together form a -CH= bridge and the bond that links it to the aromatic ring is a single bond, and in that case A represents a CH₂ group and RA represents a hydrogen atom, or a hydroxy or alkoxy group located in the 5-position of the aromatic ring carrying it,
. or RB and RC together form a single bond and A then represents a group 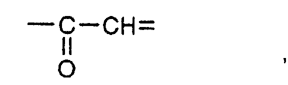 the carbonyl being linked to the oxygen and the bond joining A to the carbon carrying the side chain being a double bond, and in that case RA represents a hydrogen atom or a hydroxy or alkoxy group,
- when RB represents an alkyl group, X and Y each represent two hydrogen atoms or form together, with the carbon atom carrying them, a C=O group, and RD represents a hydrogen atom or an alkyl group,
- when RB and RC form a bridge, X and Y each represent two hydrogen atoms . and RD, which does not exist unless all the bonds of the carbon atom carrying it are single bonds, represents a hydrogen atom,
wherein, unless indicated to the contrary,
- the terms "alkyl" and "alkoxy" denote linear or branched saturated groups containing from 1 to 6 carbon atoms,
- the term "substituted" associated with the substituents phenyl and naphthyl means that those substituents may be substituted by from one to three groups selected from hydroxy, alkyl, alkyl substituted by one or more halogen atoms, alkoxy and halogen,
their optical isomers in pure form or in the form of a mixture, and addition salts thereof with a pharmaceutically acceptable acid or base.

2. Compound of formula (I) according to claim 1 which is 1-[(5-fluoro-2,3-dihydrobenzofur-2-yl)methyl]-4-benzylpiperazine.

3. Compound of formula (I) according to claim 1 which is 1-[(2,3-dihydrobenzofur-2-yl)methyl]-4-benzylpiperazine.

4. Compound of formula (I) according to claim 1 which is 1-[(chroman-2-yl)methyl]-4-benzylpiperazine.

5. Compound of formula (I) according to claim 1 which is 1-[(7-methoxy-[2H]-2-oxochrom-3-en-4-yl)methyl]-4-benzylpiperazine.

6. Compound of formula (I) according to claim 1 which is 1-[(7-methoxy-[2H]-2-oxochrom-3-en-4-yl)methyl]-4-(4-methoxybenzyl)piperazine.

7. Compound of formula (I) according to claim 1 which is 1-[2-(phenoxy)-propyl]-4-benzylpiperazine.

8. Process for the preparation of compounds of formula (I) according to claim 1, characterised in that a compound of formula (II): 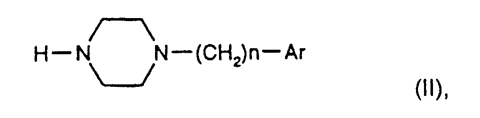 in which Ar and n are as defined in claim 1,
is reacted
- either with a compound of formula (III): 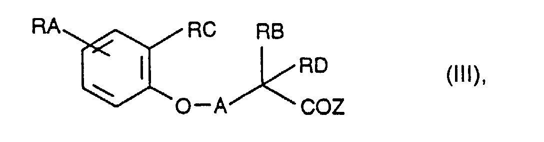 in which RA, RB, RC, RD and A are as defined in claim 1 and Z represents a hydroxy group or a chlorine atom,
to obtain a compound of formula (la): 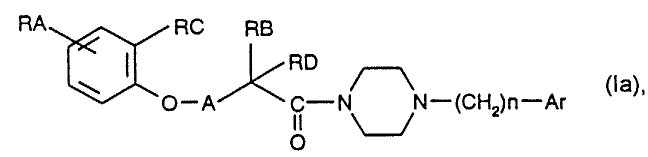 in which RA, RB, RC, RD, Ar, n and A are as defined above, which compound (la) may be subjected, in dependence upon the structure of the compound of formula (I) which it is desired to obtain, to reduction to yield a compound of formula (Ib): 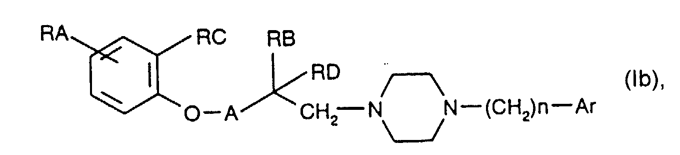 in which RA, RB, RC, RD, Ar, n and A are as defined above,
- or with a compound of formula (IV) in the presence of an alkaline agent: 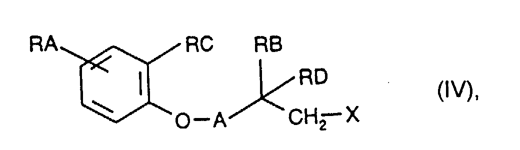 in which RA, RB, RC, RD and A are as defined above and X represents a halogen atom, to obtain a compound of formula (Ib) as defined above, the compounds of formulae (la) and (Ib) forming the totality of the compounds of formula (I) according to claim 1, which can be separated into their various optical isomers in pure form or in the form of a mixture and converted into a salt with a pharmaceutically acceptable acid or base.

9. Pharmaceutical compositions comprising as active ingredient at least one compound according to any one of claims 1 to 7 in combination with one or more pharmaceutically acceptable excipients.

10. Pharmaceutical compositions according to claim 9 for use in the prevention or treatment of disorders of the central nervous system and in inflammatory diseases of immune origin.

## Patentansprüche

1. Verbindung der Formel (I): 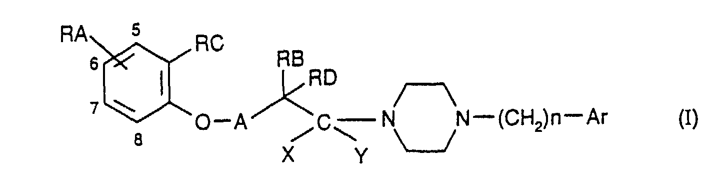 in der
- Ar eine Phenyl-, Naphthyl-, substituierte Phenyl- oder substituierte Naphthylgruppe darstellt,
- n eine ganze Zahl mit einem Wert zwischen 1 und 4 einschließlich bedeutet,
. RB eine Alkylgruppe darstellt, und in diesem Fall A eine Einfachbindung und RA und RC, die gleichartig oder verschieden sind, unabhängig voneinander ein Wasserstoffatom oder eine Gruppe ausgewählt aus Halogen, Alkyl, durch ein oder mehrere Halogene substituiertem Alkyl und Alkoxy darstellen,
. oder RB und RC gemeinsam eine Brücke -(CH₂)_{q}- bilden, worin q einen Wert von 0, 1 oder 2 besitzt und in diesem Fall A eine Brücke-(CH₂)ₚ darstellt, worin p 0, 1 oder 2 darstellt und p + q 1 oder 2 bedeuten, wobei in diesem Fall RA eine Hydroxygruppe oder Alkoxygruppe in der 5-Stellung des sie tragenden aromatischen Kerns bedeutet oder RA ein Wasserstoffatom oder ein Halogenatom in irgendeiner Stellung des aromatischen Rings bedeutet,
. oder RB und RC gemeinsam eine Brücken -CH= bilden, wobei die Bindung, die sie mit dem aromatischen Ring verbindet, einfach ist und wobei in diesem Fall A eine Gruppe CH₂ und RA ein Wasserstoffatom, eine Hydroxygruppe oder eine Alkoxygruppe in der 5-Stellung des sie tragenden aromatischen Rings bedeuten,
. oder RB und RC gemeinsam eine Einfachbindung darstellen und in diesem Fall A eine Gruppe 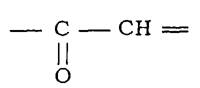 bedeutet, wobei die Carbonylgruppe an das Sauerstoffatom gebunden ist und die Bindung, über welche A mit dem die Seitenkette tragenden Kohlenstoffatom verbunden ist, eine Doppelbindung ist und in diesem Fall RA ein Wasserstoffatom oder eine Hydroxy- oder Alkoxygruppe darstellt,
- wenn RB eine Alkylgruppe darstellt, X und Y zwei Wasserstoffatome bedeuten oder gemeinsam mit dem sie tragenden Kohlenstoffatom eine Gruppe C=O bilden und RD ein Wasserstoffatom oder eine Alkylgruppe darstellt,
- wenn RB und RC eine Brücke bilden, X und Y zwei Wasserstoffatome bedeuten, und RD, welche nur existiert, wenn sämtliche Bindungen des sie tragenden Kohlenstoffatoms Einfachbindungen sind, ein Wasserstoffatom darstellt,
mit der Maßgabe, daß, wenn nichts anderes angegeben ist
- die Begriffe "Alkyl" und "Alkoxy" für geradkettige oder verzweigte gesättigte Gruppen, die 1 bis 6 Kohlenstoffatome enthalten, stehen,
- der Begriff "substituiert", wie er für die Substituenten von Phenyl und Naphthyl benutzt wird, bedeutet, daß diese durch eine bis drei Gruppen ausgewählt aus Hydroxy, Alkyl, durch ein oder mehrere Halogene substituiertem Alkyl, Alkoxy und Halogen substituiert sein können,
deren optische Isomere in reiner Form oder in Form einer Mischung und deren Additionssalze mit einer pharmazeutisch annehmbaren Säure oder Base.

2. Verbindung der Formel (I) nach Anspruch 1, nämlich 1-[(5-Fluor-2,3-dihydrobenzofur-2-yl)-methyl]-4-benzylpiperazin.

3. Verbindung der Formel (I) nach Anspruch 1, nämlich 1-[(2,3-Dihydrobenzofur-2-yl)-methyl]-4-benzylpiperazin.

4. Verbindung der Formel (I) nach Anspruch 1, nämlich 1-[(Chroman-2-yl)-methyl]-4-benzylpiperazin.

5. Verbindung der Formel (I) nach Anspruch 1, nämlich 1-[(7-Methoxy-[2H]-2-oxochrom-3-en-4-yl)-methyl]-4-benzylpiperazin.

6. Verbindung der Formel (I) nach Anspruch 1, nämlich 1-[(7-Methoxy-[2H]-2-oxochrom-3-en-4-yl)-methyl]-4-(4-methoxybenzyl)-piperazin.

7. Verbindung der Formel (I) nach Anspruch 1, nämlich 1-[2-(Phenoxy)-propyl]-4-benzylpiperazin.

8. Verfahren zur Herstellung der Verbindungen der Formel (I) nach Anspruch 1, dadurch gekennzeichnet, daß man eine Verbindung der Formel (II): 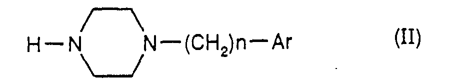 in der Ar und n die in Anspruch 1 angegebenen Bedeutungen besitzen,
- entweder mit einer Verbindung der Formel (III): 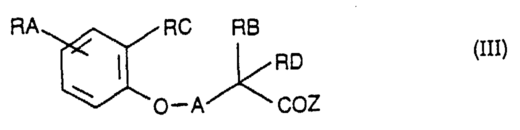 in der RA, RB, RC, RD und A die in Anspruch 1 angegebenen Bedeutungen besitzen und Z eine Hydroxygruppe oder ein Chloratom darstellt,
umsetzt zur Bildung einer Verbindung der Formel (Ia): 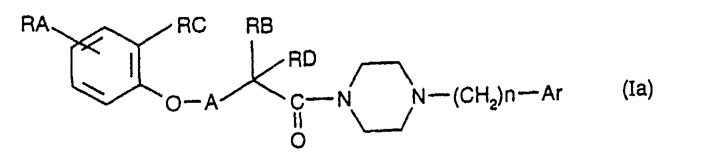 in der RA, RB, RC, RD, Ar, n und A die oben angegebenen Bedeutungen besitzen, welche Verbindung (Ia) in Abhängigkeit von der herzustellenden Verbindung der Formel (I) einer Reduktion unterworfen werden kann zur Bildung einer Verbindung der Formel (Ib): 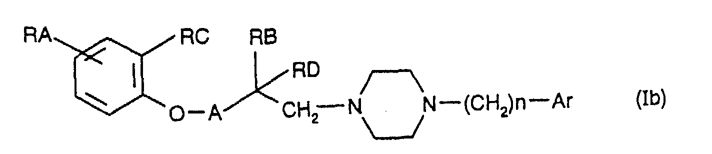 in der RA, RB, RC, RD, Ar, n und A die oben angegebenen Bedeutungen besitzen,
- oder mit einer Verbindung der Formel (IV): 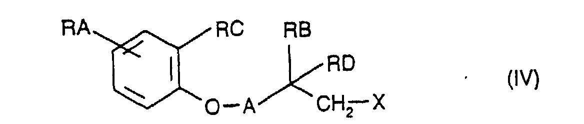 in der RA, RB, RC, RD und A die oben angegebenen Bedeutungen besitzen und X ein Halogenatom darstellt, in Gegenwart eines alkalischen Mittels umsetzt zur Bildung einer Verbindung der Formel (Ib), wie sie oben definiert worden ist, welche Verbindungen der Formeln (Ia) und (Ib), die die Gesamtheit der Verbindungen der Formel (I) nach Anspruch 1 bilden, in ihre verschiedenen optischen Isomeren in reiner Form oder in Form einer Mischung getrennt und mit einer pharmazeutisch annehmbaren Säure oder Base in ihre Salze überführt werden können.

9. Pharmazeutische Zubereitungen enthaltend als Wirkstoff mindestens eine Verbindung nach einem der Ansprüche 1 bis 7 in Kombination mit einem oder mehreren pharmazeutisch annehmbaren Trägermaterialien.

10. Pharmazeutische Zubereitungen nach Anspruch 9 zur Vorbeugung oder Behandlung von Störungen des Zentralnervensystems sowie Entzündungserkrankungen immunitären Ursprungs.
